# EUROPEAN PATENT APPLICATION

(11) **EP 3 015 056 A1**
(43) Date of publication of application: **04.05.2016**
(21) Application number: 15002968.4
(22) Date of filing: 19.10.2015
(51) Int. Cl.: A61B 5/00, A61B 5/11, A61B 5/06

(54) **THE METHOD OF EVALUATION OF THE CERVICAL SPINE DYSFUNCTION**

(30) Priority: 30.10.2014 PL 40999414
(71) Applicant: Politechnika Lódzka, 90-924 Lódz (PL)
(72) Inventor: Kujawa, Jolanta, 93-377 Lódz (PL); Napieralski, Andrzej, 95-073 Jedlicze B (PL); Adamczewski, Tomasz, 92-252 Lódz (PL); Sakowicz, Bartosz, 93-534 Lódz (PL); Kaminski, Marek, 93-150 Lódz (PL); Ritter, Robert, 92-508 Lódz (PL); Mielczarek, Aleksander, 94-333 Lódz (PL)
(74) Representative: Kaczur-Kaczynska, Ewa

(57) **Abstract**

The method of evaluating the dysfunction of the cervical spine by mounting on the patient's head a measuring device for determining the position of the head relative to the trajectory that the patient is expected to reproduce by the movement of his head, where the measuring device is connected via an interface to the expert system on a computer. Then the calibration of the data acquisition module of the expert system is started and the patient, using a laser pointer mounted as well on his head indicates the calibration points displayed on a computer monitor or projector connected to a computer, then the points are allocated in memory of the data acquisition module of the expert system to the given location of the patient's head relative to the calibration points, forwarded by the measuring device placed on the patient's head. Subsequently, the screen with the expected head movement trajectory is selected from among boards with head movement trajectories stored in the memory of the test preparation module of the expert system corresponding to a condition of the patient and the patient is examined by trying to follow the trajectory displayed on a computer monitor or projector with a computer with his head's movement. The trajectory of the patient's head, taken by the laser pointer, is introduced in form of signals from the measuring device containing the patient's head position during the examination, by means of the data acquisition module of the expert system to the data analysis module of the expert system comparing the trajectory given to the trajectory retrieved from the patient's head movement and reads on the monitor of the computer, given by the presentation of the test results module of the expert system, the examination results in form of quantity and quality of mistakes made by the patient when trying to reproduce the trajectory by moving his head, for the determination of the probability of disease of the cervical spine and choose the method of rehabilitation.

## Description

The invention presented herein is a method of assessing the dysfunction of the cervical spine. This method will be used in physiotherapy to classify patients for cervical spine dysfunction and also to support and monitor the process of their rehabilitation.

There is a known solution on how to diagnose and rehabilitate the cervical spine, developed by the company Nordisk AS, called Sensoneck. In the process a paper board is used with different lines, of a thickness of 2 cm, plotted on it: the ellipse, the lemniscate, two straight lines intersecting at an angle of 60° and two straight lines intersecting at an angle of 90°. The board's dimensions are 120 x 80 cm. In order to make the examination easier, each of the figures is drawn in a different color. To diagnose and rehabilitate the patient, he has to take place in front of the paper board, so that at the height of his eyes is at the center of the table. A laser pointer is fixed to the patients head using a band, so that it permanently points at the place on a board on which the patient looks at during the study. He tries to replicate the shapes indicated by the physiotherapist by moving his head only. The correctness of execution of the exercise is monitored by a physiotherapist who continuously assesses the degree of functional disorders of the cervical spine by estimating the amount and amplitude of errors and their dependence on the speed of the movement and the duration of the test.

The method for assessing the dysfunction of the cervical spine, based on a patient's attempt to draw with a laser pointer attached to his head, only by the head movement, the trajectory indicated on the board placed at the height of his sight and comparing the trajectory of the head movement made visible by the laser pointer trajectory indicated on the board, **according to the invention** is characterized in that, on the patient's head in addition to the laser pointer is fixed a measuring device for determining the position of the head relative to the trajectory given to him to reproduce by the movement of the head, connected by an interface to a computer's expert system module comprising of: patient adding and selection module, test preparation module, test data acquisition module communicating with the measuring device placed on the patient's head, data analysis module, test results presentation and analysis module and data storage module. When the calibration of the data acquisition module expert system is started, the patient using a laser pointer mounted on his head indicates the calibration points displayed on a computer monitor or projector connected to a computer. These points are then assigned in the data acquisition module's memory of the expert system to the data of the patient's head position relative to the calibration points, forwarded by the measuring device placed on the patient's head. A board with the trajectories of head movement adapted to study various diseases of the cervical spine is then selected from a set stored in the memory of the test preparation module of the expert system. The board with the trajectory of head movement, corresponding to a examination required for given patient is shown and the patient is trying to trace the trajectory displayed on a computer monitor or projector connected to a computer by moving his head. The recorded trajectory of the patient's head movement, tracked using a laser pointer, is sent, in the form of readouts from the measuring device telling about the location of the head of the patient during the process of reproducing the trajectory, using the data acquisition module of expert system to the data analysis system module comparing the reference trajectory to the trajectory recorded by the patient's head movement and presented on a computer screen, provided by the test results' demonstration module of the expert system. The results of the examination, such as the quantity and quality of mistakes made by the patient when trying to reproduce the trajectory with his head, are used for the determination of the probability of disease of the cervical spine and choosing the method of rehabilitation. Before a patient can be examined, his personal information has to be entered to the patient addition and selection module of the expert system and subsequently to the storage module of the expert system. The subsequent examinations of the same patient performed using the same trajectory, assuming that his personal information was introduced to the patient addition and selection module of the expert system beforehand, after performing another examination can be used to present on the computer screen the results of the last study and at the same time trends in the accuracy of mapping the patient's head movement trajectory compared to studies carried out earlier, which enables the assessment of the patient's rehabilitation methods. The measuring device, mounted on the patient's head, is the apparatus comprising of accelerometer, magnetometer, gyroscope and a module to transmit data via an interface to a computer, all placed on a single PCB.

The presented invention is an automated method of assessing the dysfunction of the cervical spine and allows the transfer of responsibility for monitoring exercises performed by the patient from the physiotherapist on to the computer. The additionally used measuring device allows to determine the position of the patient's head with respect to the displayed trajectory with an accuracy of 1 mm. It allows to adjust the trajectory of head movement to a specific disease and to monitor changes in the cervical spine dysfunction over time and thus choosing the appropriate method of rehabilitation. Courses of study based on selected trajectories are stored in memory of the expert system and can be read from memory and presented on a computer monitor or projector. All studies of a single patient undergo automatic comparative analysis, which allows for assessment of the progress or lack of progress in the rehabilitation of the patient. It allows to define the patient by giving its identification data (patient identification data can be stored in encrypted form). Using a special add-in for the physiotherapist it is possible to prepare a trajectory chosen individually for the condition of the patient. A stripped down version of the expert system, complemented by the individual trajectory can be provided to the patient for rehabilitation at home. The patient, after executing the exercises, can export the results and transfer them to his doctor during consultation.

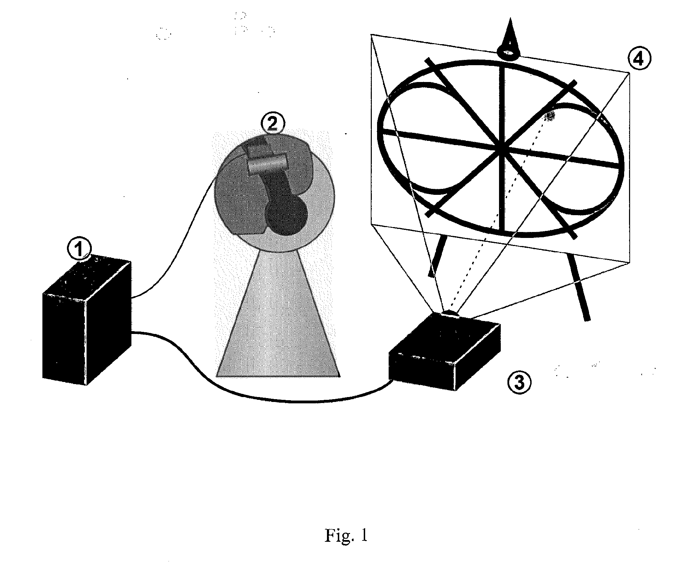

The invention is illustrated in the following example with reference to the drawing showing in a perspective view a system for carrying out the process.

### Example.

To evaluate the dysfunction of the cervical spine a system comprising of a computer 1, which input is connected to the measuring device 2 with a built-in laser pointer, and the output is a multimedia projector 3 displaying screen 4 is used. The computer 1 is provided with an expert system module comprising the patient adding and selection module, test preparation module, test data acquisition module, data analysis module, the test results demonstration and analysis module and data storage module. The patient selection and addition module provides a set of text and choice fields, enabling the introduction of patient's identification data and allowing the search and selection of a patient based on the identification number. The test preparation module contains a form which allows to choose the board used during the examination and allows to start the calibration, examination and to complete the test. It is also responsible for displaying the selected board for examination. The data acquisition module communicates with the measuring device 2, reads the output from the measurement device and displays it on the board and simultaneously transmits the position to the data storage module. The data storage module consists of a free and publicly available database engine and a communication interface for other expert system modules. The data analysis module includes measurement data analysis algorithms and communication interface for measurement data storage unit and module demonstration of the results of research and analysis. The results of research and analysis demonstration module consists of a screen that shows a list of studies conducted for the selected in the selection and adding patient module and the screen showing the selected conducted research together with a statistical evaluation of the patient's ailment.

A patient that was defined previously at the first test, came in for the study. The process of patient record definition requires that the person operating the expert system passes to the patient addition and selection module the patient identification data. These consists of patient identity, composed of the first name, last name, date of birth, identification number and gender. After approval, the data are saved in memory and then are transmitted to data storage module of the expert system.

An expert system operator, after providing the identification number of the predefined patient to the storage module of the expert system finds the patient in the database and selects him for the examination. After the patient is chosen, all the test results are associated with his person. In order to perform the examination the patient places the measuring device 2, placed in a holder and connected to a computer 1 running the expert system, on his head. Then, the operator of the expert system, using the test preparation module, selects the type of examination and starts the calibration process of device 2, which means determining the extreme positions of the head relative to the board. On the screen 4 of the monitor which is displayed by multimedia projector 3 is shown the center point of the board to which the patient must point in the resting position. The task of the expert system operator is to shift the board and adjust it to the patient's position so that he has the center of the board perfectly on line of sight. Then, using the calibration interface visible to the operator, calibration points are selected. The patient must direct his head toward the calibration point and indicate with the laser pointer built into the device 2 its center. The person operating the system, after the patient's pointing the calibration point, chooses to display the next calibration point. Data acquisition module records data from the device 2 for the point indicated by the patient and displays the next calibration point on the screen 4. When calibration is complete, the preselected trajectory is displayed on the screen 4 and movement of the patient's head is recorded by the data acquisition module of the expert system. The patient reproduces the trajectory by moving his head during the examination. Each head movement is recorded by the data acquisition module into memory as part of the examination, then it goes to the data storage module as part of the whole examination. The operator of the expert system watches the development of the examination of the patient, and after mapping the entire trajectory by the patient, the operator selects from the interface of the study preparation module of the expert system, visible only to him, the end of the examination. At this point, a full course of study goes to the data storage module, from where it is then downloaded by the data analysis module and analyzed for quantity and quality of committed errors, and because previous test data was available, the change for the same trajectory in subsequent approaches is assessed (improvement, deterioration) and probability of occurrence of diseases of the cervical spine is calculated. This information is stored into the database of the storage module and is associated with the patient and this particular trajectory.

Then, the operator introduces to the patient adding and selecting module the patient's identification number, and after searching the patient chooses from a list of examinations of the demonstration of the results of research and analysis module previously conducted study. It appears on the computer screen 1 on top of the selected trajectory during the test. Through selecting from the carried out studies on the same trajectory, changes in the performance of the examination of the selected patient are presented. The expert system automatically identifies tendencies in the accuracy of mapping the trajectory by the patient's head movement.

## Claims

1. A method for assessing the dysfunction of the cervical spine, consisting in an attempt to redraw by the patient equipped with a laser pointer attached to his head, by head movement, the trajectory indicated on a board placed at the height of the patient's sight and compared the trajectory of his head being tracked by a laser pointer to the trajectory indicated on the board, **characterized in that** on the patient's head is additionally fixed a measuring device for determining the position of the head relative to the trajectory given to him to redraw by the movement of the head, connected by an interface to an expert system of the computer, that contains: patient addition and selection module, test data preparation module, data acquisition module communicating with the measuring device placed on the head of the patient, data analysis module, test results demonstration and analysis module, data storage module, then starts the calibration of the data acquisition module of the expert system and the patient, using the laser pointer mounted at its head indicates the calibration points displayed on a computer monitor or projector connected to a computer, then the points are allocated in memory of the data acquisition module of the expert system to the given location of the patient's head relative to the calibration points, forwarded by the measuring device placed on the patient's head, then chosen from among stored in the memory of the test preparation module of the expert system boards with the trajectories of head movement suited to the study of various diseases of cervical spine, the board with the trajectory of head movement corresponding to a condition examined by the patient is taken while the patient by moving his head is trying to follow the trajectory displayed on a computer monitor or projector connected to a computer, then the trajectory made from the head's movement of the patient tracked using a laser pointer, is introduced in the form of signals from measuring device containing information about the location of the patient's head during redraw of the trajectory, using the data acquisition module of the expert system to the data analysis module of the expert system comparing the trajectory set to the trajectory made by the movement of the head of the patient and read on a computer screen, provided by the test results demonstration module of the expert system, the survey findings as the quantity and quality of mistakes made by the patient when trying to redraw the trajectory with his head's movement, for the determination of the probability of disease of the cervical spine and choosing the method of rehabilitation, while before a new patient is examined he is defined by entering personal data to the addition and patient selection module of the expert system, and subsequently to the data storage module of the expert system, and during the subsequent examinations of the patient made using the same trajectory before the test patient's data is introduced to the addition and patient selection module of the system expert and after examination on a computer screen watching the results of the study and also trends in correctness of mapping the patient's head movement in relation to tests performed before, which enables the assessment of the patient's rehabilitation methods.

2. Method according to claim 1, **characterized in that** the measuring device attached to the patient's head is a device comprising of an accelerometer, magnetometer and a gyroscope placed on a single printed circuit board together with a module for transferring data to a computer via an interface.
